# EUROPÄISCHE PATENTSCHRIFT

(11) **EP 0 431 349 B1**
(45) Veröffentlichungstag und Bekanntmachung des Hinweises auf die Patenterteilung: **29.03.1995**
(21) Anmeldenummer: 90121549.1
(22) Anmeldetag: 10.11.1990
(51) Int. Cl.: C07C 53/48, C07C 51/62

(54) **Verfahren zur Herstellung von Trichloracetylchlorid**
Process for preparing trichloroacetyl chloride
Procédé de préparation de chlorure de trichloracétyle

(30) Priorität: 15.11.1989 DE 3937981
(43) Veröffentlichungstag der Anmeldung: 12.06.1991
(73) Patentinhaber: HOECHST AKTIENGESELLSCHAFT, 65926 Frankfurt am Main (DE)
(72) Erfinder: Freyer, Walter, Dr., W-8901 Leitershofen (DE); Miltenberger, Karlheinz, Dr., W-8906 Gersthofen (DE); Schmidt, Manfred, W-8870 Günzburg-Deffingen (DE)

(56) Entgegenhaltungen:
- US-A- 3 751 461
- JOURNAL OF GENERAL CHEMISTRY USSR. Bd. XXVI, 1956, NEW YORK US Seiten 451 - 454; B. A. PORAI-KOSHITS ET AL.: 'The Chlorination Of Ketene'

## Beschreibung

Die Erfindung bezieht sich auf ein Verfahren zur Herstellung von Trichloracetylchlorid, bei welchem Dichloracetylchlorid in Gegenwart eines Katalysators chloriert wird.

Überlicherweise wird Trichloracetylchlorid durch Oxidation von Tetrachlorethen unter Einwirkung von kurzwelligem Licht hergestellt. Das so gewonnene Säurechlorid enthält jedoch geringe Mengen des Ausgangsproduktes, die sich wegen der sehr nahe beieinanderliegenden Siedepunkte durch Destillation nur unter sehr großem Aufwand abtrennen lassen. Das rohe Säurechlorid läßt sich zwar in einigen Fällen direkt weiterverarbeiten, beispielsweise zu den Estern, welche sich leichter vom Tetrachlorethen trennen lassen, dieses Säurechlorid ist jedoch nicht für alle Einsatzzwecke geeignet.

Es wurde nun gefunden, daß man ein reineres Trichloreacetylchlorid, welches sich leichter von den Verunreinigungen abtrennen läßt, gewinnen kann, wenn man Dichloracetylchlorid in Gegenwart eines Katalysators chloriert.

Bekannt ist die Chlorierung von Chloracetylchlorid unter Verwendung von Pyridin als Katalysator (vgl. Journal of General Chemistry USSR, Bd. XXVI, 1956, 451-454). Nach Beendigung der Reaktion werden 30 % des eingesetzten Chloracetylchlorids zurückerhalten sowie 5 % Dichloracetylchlorid und 65 % Trichloracetylchlorid. Dieses Ergebnis wird von den Autoren dahingehend interpretiert, daß in Gegenwart von Pyridin Dichloracetylchlorid leichter mit Chlor reagiere als das ursprünglich eingesetzte Chloracetylchlorid. Die Chlorierung von Dichloracetylchlorid in Gegenwart von Pyridin unter Bildung von Trichloracetylchlorid wird demzufolge nicht als eigenständige Reaktion unter definierten Reaktionsbedingungen durchgeführt.

Bekannt ist weiterhin die Chlorierung von Säurechloriden unter Einsatz von Alkylpyridinen oder Tetraalkylammoniumhalogeniden als Katalysatoren (vgl. US-A 3,751,461). Als verwendbare Säurechloride werden neben den reinen Kohlenwasserstoff-Säurechloriden auch substitutierte Säurechloride aufgeführt, die in α-Position mindestens ein H-Atom enthalten. Desweiteren wird offenbart, daß der Katalysator dem Säurechlorid jeweils in einer Menge von 1 bis 10 Gew. % zugesetzt wird.

Die Erfindung betrifft somit das in den Ansprüchen beschriebene Verfahren.

Das erfindungsgemäße Verfahren wird in der Weise durchgeführt, daß Chlor bei erhöhter Temperatur mit Dichloracetylchlorid in Berührung gebracht wird.

Für das Verfahren sind korrosionsfeste Rührgefäße geeignet, welche im Verhältnis zur Menge des eingesetzten Dichloracetylchlorids ausreichend groß sind, da der Ansatz zum Schäumen neigt. Vorteilhaft ist intensives Mischen des Ansatzes. Brauchbar sind auch Reaktoren, in denen ein flüssiger dünner Film eines Produktes mit einem Gas reagieren kann, insbesondere für den kontinuierlichen Betrieb. Auf jeden Fall muß sichergestellt sein, daß das entstehende HCl-Gas ungehindert entweichen kann. Die Reaktionsgefäße sind mit den üblichen Einrichtungen zum Heizen, Kühlen und Mischen sowie zum Messen und Regeln ausgestattet.

Die Reaktion wird bei einer Temperatur von 90 bis 120°C, vorzugsweise 100 bis 110°C, in Gegenwart eines Katalysators durchgeführt. Dieser Katalysator ist ein tertiäres Amin oder ein C₅- oder C₆-N-Heterocyclus, vorzugsweise C₆-N-Heterocyclus, insbesondere Pyridin oder Picolin, und wird in einer Menge von 0,08 bis 0,14 Gew.-%, bezogen auf das eingesetzte Dichloracetylchlorid, angewendet.

Nach Beendigung der Umsetzung, die zweckmäßigerweise mittels eines Gaschromatografen überwacht wird, wird das überschüssige Chlor entfernt, beispielsweise durch Durchleiten eines Inertgases. Danach kann das Trichloracetylchlorid gegebenenfalls durch fraktionierte Destillation auf einen höheren Reinheitsgrad gebracht werden.

Die Ausbeute beträgt mehr als 90 Gew.-%, bezogen auf eingesetztes Dichloracetylchlorid.

Die nachfolgenden Beispiele sollen die Erfindung näher erläutern.

### Beispiel 1

In einem 1-dm³-4-Halskolben mit Innen- und Übergangsthermometer, Gaseinleitrohr, Intensivkühler mit Gasabgang wurden 589,6 g Dichloracetylchlorid (DAC) (4 mol) vorgelegt und mit 0,6 g Pyridin (0,1 %) unter Rühren versetzt. Das mit Pyridin versetzte DAC wurde im Ölbad auf 98-107 °C aufgeheizt und unter schnellem Rühren chloriert. Anfänglich kam es dabei zu starken Schäumen des Produktes. Gegen Ende der Chlorierung hörte das Schäumen auf. Der Stand der Umsetzung wurde gaschromatografisch verfolgt. Nach 4¾ h war das DAC nahezu quantitativ zu TAC chloriert. Das Produkt war chlorhaltig und leicht gelb gefärbt. Zur Entfernung des überschüssigen Chlors wurde ca. ½ h Luft bzw. N₂ durch das TAC geleitet. Danach war das TAC nur noch ganz schwach gelb gefärbt. Die Ausbeute betrug ca. 92 %. Die Chlorierung erfolgte nur in Gegenwart eines Katalysators. Bei Chlorierungsversuchen von DAC ohne Zusatz wurde keine Chloraufnahme festgestellt. Der Ersatz von Pyridin durch Diethanolamin zeigte keine katalytische Wirkung.
Zur Bestimmung der optimalen Pyridinmenge wurden Chlorierungen mit Pyridineinsätzen von 0,05 bis 0,5 % durchgeführt. Als optimale Pyridinmenge wurde ca. 0,1 % ermittelt.
Ebenso wurde auch die Chlorier-Temperatur variiert. Die kürzeste Chlorierungszeit wurde nahe am Siedepunkt des Reaktionsgemisches (Rückflußtemperatur) erzielt.

### Beispiel 2

590 g 97 %iges Dichloracetylchlorid wurden in Gegenwart von 0,1 % Pyridin 8 h chloriert. Dabei wurden 663 g Trichloracetylchlorid 96,3 %ig erhalten. Das entspricht einer Ausbeute von ca. 91 %, wobei durch Probennahmen Verluste auftraten.
Während der Chlorierung wurden stündlich Proben genommen und hinsichtlich der TAC-Bildung gaschromatografisch untersucht. Auch das bei der Chlorierung entstehende HCl-Gas wurde in den ersten beiden Waschflaschen, die mit dest. Wasser gefüllt waren, aufgefangen und stündlich mit AgNO₃-Lösung titriert.
Die gaschromatografisch gefundene TAC-Zunahme (bzw. DAC-Abnahme) und die titrimetrisch ermittelten HCl-Werte sind in Tabelle 1 in Abhängigkeit von der Zeit zusammengefaßt. Die ermittelten Chlor-Werte in den einzelnen Waschflaschen sowie ihre Gesamtmenge sind in Tabelle 2 zusammengestellt. Da in der letzten, Natronlauge enthaltenden Wasserflasche kein Chlor mehr gefunden wurde, ist sichergestellt, daß sämtliches überschüssiges Chlor erfaßt wurde. Insgesamt haben sich bei dem Versuch 40 g Chlor nicht umgesetzt, das sind 13,7 % des eingesetzten Chlors. Die Versuchsdauer betrug 7½ h.

### Beispiel 3

Zur Minimierung der Chlormenge wurden weitere Versuche durchgeführt, bei denen die Chlorzugabe nach und nach reduziert wurde. Das dabei anfallende Abgas bestand aus HCl-Gas und überschüssigem Chlor. Es wurde in Waschflaschen geleitet und analysiert.
Die beiden ersten Waschflaschen dienten der HCl-Wäsche und waren jeweils mit 1 dm³ bzw. 500 cm³ Wasser gefüllt. Die beiden nachfolgenden Waschflaschen waren mit 300 g 20 %iger NaOH gefüllt, sie dienten der Chlorabsorption.
Die Chlormenge wurde solange reduziert, bis nach der letzten, Natronlauge enthaltenden Waschflasche kein Abgas mehr entwich (abgesehen von gelegentlichen Gasblasen, die temperaturbedingt waren). Da die Chlorierungsdauer stark vom Chlorangebot abhängig war, resultierte bei der niedrigsten Chlormenge die längste Chlorierungszeit. Die Versuche dauerten 4¾ h bis 8 h. Die beste Chlorausbeute wurde bei ca. 7½ h erzielt.
Die Umsetzungen wurden gaschromatographisch überwacht. Dabei ergab sich, daß die Chloraufnahme nahezu linear erfolgte. Erst gegen Ende flachte sie langsam ab.

**Tabelle 1**

| Gaschromatographische Bestimmung der TAC-Bildung (bzw. DAC-Abnahme) und titrimetrische Ermittlung der HCl-Entwicklung in Abhängigkeit von der Zeit. | | | | | |
|---|---|---|---|---|---|
| | GC-Werte | | HCl-Bildung | | |
| Zeit Std. | % DAC Abnahme | % TAC Zunahme | Cl⁻ 1. Waschfl. | Cl⁻ 2. Waschfl. | Summe Cl⁻ |
| 1 | - | - | 16,7 g | 0 g | 16,7 g |
| 2 | 73,3 | 24,6 | 37,5 g | 0,9 g | 38,4 g |
| 3 | 65,2 | 33,0 | 50,8 g | 1,1 g | 51,9 g |
| 4 | 45,9 | 51,5 | 72,8 g | 1,3 g | 74,1 g |
| 5 | 34,1 | 63,8 | 94,5 g | 1,5 g | 96,0 g |
| 6 | 19,6 | 78,6 | 104,5 g | 1,5 g | 106,0 g |
| 7 | 7,0 | 90,4 | 120,0 g | 1,0 g | 121,0 g |
| 8 | 0,5 | 96,3 | 128,6 g | 1,8 g | 130,4 g |

**Tabelle 2**

| Zeitliche Bestimmung des Chlorüberschusses durch Titration in den nachgeschalteten Waschflaschen. | | | | | |
|---|---|---|---|---|---|
| | Chlor-Überschuß | | | | |
| Zeit Std. | 1. Waschfl. Wasser freies Cl₂ | 2. Waschfl. Wasser freies Cl₂ | 3. Waschfl. NaOH freies Cl₂ | 3. Waschfl. NaOH Cl⁻ | gesamte durchgeschlagene Chlor-Menge |
| 1 | 0,2 g | 0,3 g | 0 g | 0 g | 0,5 g |
| 2 | 2,4 g | 2,8 g | 0,5 g | 1,1 g | 6,8 g |
| 3 | 1,9 g | 2,1 g | 0 g | 5,9 g | 9,9 g |
| 4 | 2,5 g | 2,5 g | 0,3 g | 8,7 g | 14,0 g |
| 5 | 4,0 g | 1,7 g | 0,2 g | 19,8 g | 25,7 g |
| 6 | 2,5 g | 2,3 g | 0,1 g | 23,1 g | 28,0 g |
| 7 | 2,7 g | 1,9 g | 0,1 g | 29,0 g | 33,7 g |
| 8 | 1,4 g | 2,1 g | 0,5 g | 36,7 g | 40,7 g |

### Beispiel 4

Beispiel 2 wurde wiederholt, jedoch wurde als Katalysator α-Picolin in einer Menge von 0,1 % eingesetzt. Man erhielt 95,66 % Trichloracetylchlorid und 3,30 % Dichloracetylchlorid.

## Patentansprüche

1. Verfahren zur Herstellung von Trichloracetylchlorid, dadurch gekennzeichnet, daß Dichloracetylchlorid bei einer Temperatur von 90 bis 120°C in Gegenwart eines tertiären Amins oder eines C₅-C₆-N-Heterocyclus als Katalysator chloriert wird, wobei der Katalysator in einer Menge von 0,08 bis 0,14 Gew.-%, bezogen auf das eingesetzte Dichloracetylchlorid, verwendet wird.

2. Verfahren nach Anspruch 1, dadurch gekennzeichnet, daß als Katalysator ein C₆-N-Heterocyclus verwendet wird.

3. Verfahren nach Anspruch 2, dadurch gekennzeichnet, daß als Katalysator Pyridin oder Picolin verwendet wird.

## Claims

1. Process for the production of trichloroacetyl chloride, characterized by the fact that dichloroacetyl chloride is chlorinated at a temperature of 90 to 120°C in the presence of a tertiary amine or of a C₅-C₆-N heterocyclic compound as catalyst, the catalyst being used in a quantity of 0.08 to 0.14 % by weight, relative to the dichloroacetyl chloride employed.

2. Process in accordance with Claim 1, characterized by the fact that a C₆-N heterocyclic compound is used as catalyst.

3. Process in accordance with Claim 2, characterized by the fact that pyridine or picoline is used as catalyst.

## Revendications

1. Procédé pour la préparation de chlorure de trichloroacétyle, caractérisé en ce qu'on effectue la chloration du chlorure de dichloroacétyle à une température de 90 à 120 °C, en présence d'une amine tertiaire ou d'un hétérocycle azoté en C₅-C₆ en tant que catalyseur, en utilisant le catalyseur en une quantité de 0,08 à 0,14 % en poids par rapport au chlorure de dichloroacétyle introduit.

2. Procédé selon la revendication 1, caractérisé en ce qu'on utilise en tant que catalyseur un hétérocycle azoté en C₆.

3. Procédé selon la revendication 2, caractérisé en ce qu'on utilise en tant que catalyseur la pyridine ou la picoline.
